# FASCICULE DE BREVET EUROPEEN

(11) **EP 2 071 322 B1**
(45) Date de publication et mention de la délivrance du brevet: **18.09.2013**
(21) Numéro de dépôt: 08305918.8
(22) Date de dépôt: 11.12.2008
(51) Int. Cl.: A61B 1/06, A61B 5/00, G01N 21/64

(54) **Système d'analyse par fluorescence d'un champ dans une zone éclairée**
System for analysis by fluorescence of a field in a lit zone
Analysesystem mit Hilfe der Fluoreszenz eines Felds in einer beleuchteten Zone

(30) Priorité: 11.12.2007 FR 0759744
(43) Date de publication de la demande: 17.06.2009
(73) Titulaire: Commissariat à l'Énergie Atomique et aux Énergies Alternatives, 75015 Paris (FR)
(72) Inventeur: Moy, Jean-Pierre, 38120 Saint Egreve (FR); Rizo, Philippe, 38700 La Tronche (FR); Da Silva, Anabela, 38000 Grenoble (FR)
(74) Mandataire: Thibon, Laurent

(56) Documents cités:
- WO-A-2005/110206
- DE-A1- 10 136 145
- US-A1- 2003 120 129
- US-A1- 2003 158 470
- US-A1- 2004 225 222
- US-A1- 2006 061 680
- US-A1- 2007 073 159
- US-A1- 2007 268 493
- US-B1- 6 364 829

## Description

### Domaine de l'invention

La présente invention concerne l'analyse dans un champ d'observation du rayonnement d'éléments fluorescents, ce champ d'observation étant disposé dans une zone éclairée de façon que l'on puisse observer également directement ce champ et les régions environnantes de façon naturelle.

### Etat de la technique

L'état de la technique et la présente invention vont être exposés ci-après essentiellement dans le cadre d'une application médicale ou vétérinaire de l'invention, dans lequel la zone éclairée est une salle d'opération ou autre salle d'intervention médicale ou vétérinaire, par exemple une salle d'intervention dermatologique. Le champ d'observation ou champ opératoire est alors une région du corps d'un patient dont des emplacements ont fixé des éléments fluorescents qui sont l'indication de caractéristiques physiologiques spécifiques. Par exemple, sur la peau, des zones atteintes de maladie ou attaquées par des parasites sont susceptibles de fixer plus d'éléments fluorescents que d'autres zones de la peau. Alors, une observation des éléments fluorescents (ou marqueurs) peut révéler ces zones. De même, dans un champ opératoire, une portion d'un organe atteinte de défaillance, par exemple des cellules cancéreuses, est susceptible de fixer spécifiquement des éléments fluorescents. Dans les deux cas, il faut que l'ensemble du patient et de la salle dans laquelle il se trouve soit éclairé pour que le praticien puisse observer la zone malade et éventuellement intervenir, et il faut également observer la lumière de fluorescence. Plus particulièrement dans une salle d'opération où un chirurgien doit intervenir, il importe que le champ opératoire soit particulièrement bien éclairé.

Un problème qui se pose pour l'analyse des zones fluorescentes est que, de façon générale, l'intensité lumineuse de fluorescence est très faible devant l'éclairement ambiant normal. Dans la plage de longueurs d'onde de fluorescence d'un élément fluorescent particulier, la lumière de fluorescence est souvent de 10⁶ à 10⁷ fois plus faible que la lumière ambiante dans la plage spectrale concernée. On notera que ce problème de différence d'intensité entre la lumière blanche destinée à éclairer la scène pour qu'elle soit bien visible à l'oeil nu, et la lumière de fluorescence observée par une caméra ne se pose pas dans des situations où une image en lumière blanche et une image de fluorescence sont toutes deux observées par des caméras. Dans de telles situations, par exemple en endoscopie, l'intensité de l'image résultant d'un éclairage en lumière blanche est de préférence du même ordre de grandeur que l'intensité de l'image de fluorescence.

Pour des salles d'opération, dans l'art antérieur, on a prévu de supprimer par filtrage dans la lumière ambiante la partie du spectre dans laquelle les éléments fluorescents émettent et de détecter l'image de fluorescence avec une caméra munie d'un filtre qui exclut toute lumière hors de la bande spectrale de fluorescence. Ce procédé présente de nombreux inconvénients. Le filtre doit avoir des performances exceptionnelles en termes d'extinction, car le rapport lumière ambiante/fluorescence est énorme (>10⁶) ; l'éclairage filtré perturbe gravement la vision des couleurs ; et chaque type d'élément fluorescent impose un jeu de filtres sur le système d'éclairage.

US2007073159 A1 (figures 1-3) divulgue un système d'enregistrement d'une part d'une image fluorescente d'un tissu contenant un élément fluorescent et d'autre part d'une image visible de la même région du tissu. Le système comporte des dispositifs d'illumination (1, 2) tels que les LEDs pour des éclairages respectivement dans la région infrarouge proche et dans la région visible d'un tissu (G) de sorte que une des lumières excite un colorant fluorescent dans le tissu. Le système comprend de plus un dispositif d'acquisition d'image (4) comprenant une caméra qui enregistre une image du tissu et un dispositif de synchronisation (5) qui contrôle un dispositif de traitement d'image (6) par exemple un ordinateur, de manière telle que l'image enregistrée avec la caméra est acquise séparément à l'aide du dispositif de traitement d'image, un générateur d'horloge 3, un dispositif 4 d'acquisition d'image, un dispositif 6 de traitement d'image et un écran 7. Le système est envisagé en particulier pour l'utilisation dans le cadre d'une salle d'opération par exemple pendant l'ablation chirurgicale des tumeurs.

### Résumé de l'invention

Un objet de la présente invention est de prévoir un système d'observation de lumière de fluorescence en présence d'un éclairage ambiant qui pallie au moins certains des inconvénients des systèmes antérieurs.

Un objet d'un mode de réalisation de la présente invention est de permettre l'utilisation d'un éclairement ambiant qui puisse être optimisé en termes d'intensité et de caractéristiques spectrales, indépendamment de l'analyse de la fluorescence.

Un objet d'un mode de réalisation de la présente invention est de prévoir un système qui évite le recours à des filtrages coûteux.

Un objet d'un mode de réalisation de la présente invention est de prévoir un système qui permette de choisir librement des éléments fluorescents ayant des caractéristiques optiques éventuellement très différentes.

Pour atteindre ces objets, un mode de réalisation de la présente invention prévoit un système d'analyse à l'oeil nu et par fluorescence d'un champ dans une zone éclairée selon la revendication indépendante 1 et les revendications dépendantes 2-9.

Selon un mode de réalisation de la présente invention, la fréquence d'excitation périodique est de l'ordre de 100 Hz.

Selon un mode de réalisation de la présente invention, la deuxième source lumineuse fournit de la lumière dans une région spectrale ne recouvrant pas le spectre d'émission des éléments fluorescents.

Selon un mode de réalisation de la présente invention, le dispositif d'analyse est une caméra électronique.

Selon un mode de réalisation de la présente invention, la caméra électronique comprend une matrice de pixels à transfert ligne par ligne.

Selon un mode de réalisation de la présente invention, l'image de la caméra électronique est superposée à l'image fournie par une autre caméra électronique observant ledit champ à la lumière de la première source lumineuse d'éclairement.

Selon un mode de réalisation de la présente invention, le système comprend une troisième source lumineuse de spectre sensiblement complémentaire de celui de la deuxième source lumineuse, éclairant sensiblement le même champ et active de façon complémentaire de la deuxième source lumineuse.

### Brève description des dessins

Ces objets, caractéristiques et avantages, ainsi que d'autres seront exposés en détail dans la description suivante de modes de réalisation particuliers faite à titre non-limitatif en relation avec les figures jointes parmi lesquelles :
la figure 1 représente un système d'observation de fluorescence dans un champ d'observation ;
la figure 2 représente un système d'observation de fluorescence dans un champ d'observation selon un mode de réalisation de la présente invention ;
la figure 3 est un chronogramme destiné à expliquer un mode de fonctionnement d'un système selon un mode de réalisation de la présente invention ; et
la figure 4 représente très schématiquement une portion d'une matrice de pixels utilisable selon un mode de réalisation de la présente invention.

### Description détaillée

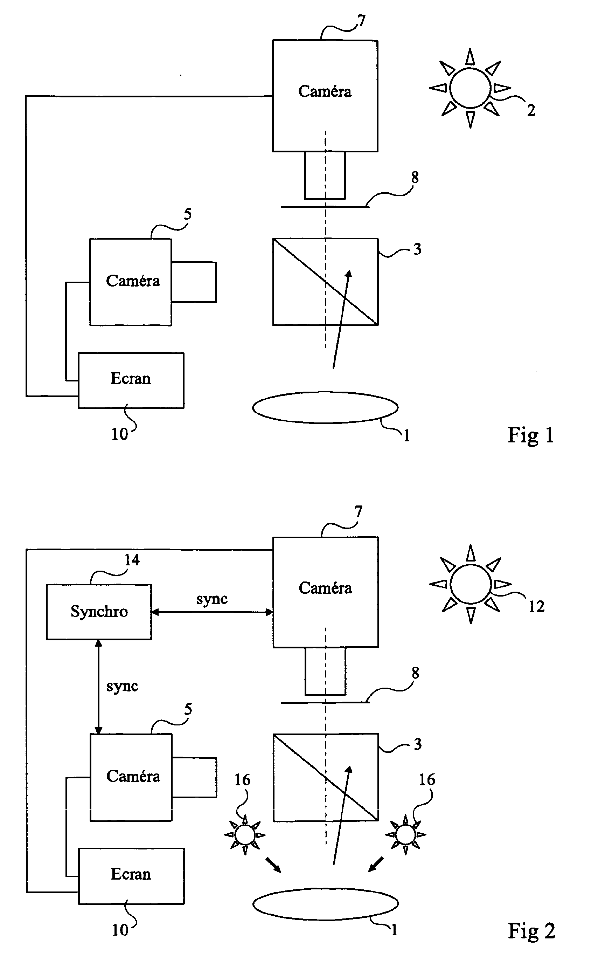

La figure 1 représente une zone à observer ou champ opératoire 1 disposé dans une salle d'opération éclairée par une source d'éclairage ambiant 2, généralement une source de lumière blanche constituée d'un ensemble de lampes à incandescence. Le champ opératoire est observé grâce à une séparatrice 3, d'une part par une caméra couleur optionnelle 5, d'autre part par une caméra 7 sensible à la seule lumière de fluorescence du champ opératoire 1. Cette lumière de fluorescence résulte par exemple d'un éclairage par une source lumineuse spécifique, non représentée. Un filtre 8 isole la plage spectrale dans laquelle est émise la lumière de fluorescence. De préférence, les images des caméras 5 et 7 sont superposées à l'aide de moyens de traitement sur un écran de visualisation 10 sur lequel on peut voir d'une part l'ensemble du champ opératoire, d'autre part les zones de ce champ plus particulièrement riches en éléments fluorescents, ces zones étant par exemple marquées d'une couleur particulière ou par des symboles spécifiques. Ainsi, un chirurgien qui observe la scène à l'oeil nu pourra en outre immédiatement voir sur l'écran 10 des zones "malades" et pourra alors les traiter ou les éliminer. Ce type d'installation se heurte aux problèmes qui ont été évoqués précédemment.

La figure 2 représente un système selon un mode de réalisation de la présente invention destiné, comme le système de la figure 1, à fournir sur un écran de visualisation 10 l'image combinée d'une caméra couleur 5 d'observation normale d'un champ opératoire et d'une caméra 7 sensible à la fluorescence, éventuellement précédée d'un filtre 8 correspondant à la plage d'émission des éléments fluorescents. Les deux caméras observent simultanément le champ opératoire 1 grâce à une séparatrice 3.

Dans le système de la figure 2, la source lumineuse d'éclairement d'ambiance est une source lumineuse 12 qui, contrairement à une lampe à incandescence, est une source lumineuse à très faible rémanence, c'est-à-dire que dès que son alimentation est interrompue (ou devient inférieure à un certain seuil) l'éclairement produit chute à une valeur nulle très rapidement, par exemple en une durée de l'ordre de quelques microsecondes. Un exemple de source lumineuse en lumière blanche à faible rémanence est constitué d'un assemblage de diodes électroluminescentes (LED) monochromes.

On notera que les sources de lumière blanche à LED ne sont généralement pas à faible rémanence. En effet, les sources de lumière blanche à LED, dites "LED blanches", sont généralement constituées d'une LED de couleur, par exemple bleue ou violette, et d'un ou plusieurs luminophore(s) externe(s) à la LED, dont le spectre d'émission se mélange avec le spectre d'émission de la LED pour produire un éclairage dont le spectre s'étend généralement entre 400 nm à au-delà de 700 nm. On obtient ainsi un éclairage dit blanc. L'inconvénient des LED blanches telles qu'elles sont actuellement réalisées, c'est-à-dire associant une LED de couleur et un luminophore, réside dans la décroissance de la lumière émise par le composé fluorescent après cessation de l'excitation, appelée rémanence. Si la décroissance de l'impulsion de lumière de couleur produite par la LED de couleur, est rapide, bien inférieure à 1 µs, la décroissance de la lumière produite par le luminophore est bien plus lente, et suit généralement une forme non-exponentielle. Il en résulte que le temps nécessaire pour que l'émission du composé fluorescent soit inférieure à 10⁻⁶ de l'émission à pleine puissance peut être très long, des dizaines ou centaines de millisecondes. Aussi, des LEDs ainsi réalisées ne constituent pas des sources de lumière à faible rémanence.

Une LED couleur à faible rémanence peut être alimentée par impulsions périodiques, et c'est d'ailleurs souvent dans ces conditions qu'elle présente le meilleur rendement lumineux. Ainsi, pour éclairer une salle d'opération, on pourra utiliser un grand nombre de diodes de couleurs différentes, dont la combinaison permettra de fournir une lumière de teinte (de couleur) réglable et particulièrement adaptée à une bonne observation, tout en conservant une très faible durée de rémanence.

Le fonctionnement de la caméra 7 sensible à la fluorescence est synchronisé par un dispositif de synchronisation 14 qui fixe la fréquence des impulsions de mise en route fournies à la source lumineuse 12. Le dispositif de synchronisation 14 active la caméra 7, et éventuellement la caméra d'observation 5, seulement pendant des durées choisies, qui seront explicitées ci-après. Une source lumineuse supplémentaire 16 éclaire le champ opératoire 1. La source lumineuse 16 a également un fonctionnement synchronisé par le dispositif 14 et permet d'éclairer le champ opératoire à une longueur d'onde d'excitation optimale pour un élément fluorescent particulier utilisé. La source lumineuse 16 est constituée d'une pluralité de sources élémentaires, par exemple des diodes électroluminescentes ou des diodes laser, éventuellement associées à un filtre optique permettant d'éliminer les longueurs d'onde correspondant à la fluorescence recherchée. Si l'élément fluorescent est de la fluorescéine, on utilisera une lumière d'excitation d'une longueur d'onde voisine de 488 nm (dans le bleu), la fluorescence étant alors à une longueur d'onde voisine de 550 nm (dans le vert). Si l'élément fluorescent est de l'Alexa 750, la lumière d'excitation aura une longueur d'onde voisine de 690 nm (dans le rouge lointain), la fluorescence étant alors à une longueur d'onde voisine de 750 nm (dans le proche infrarouge).

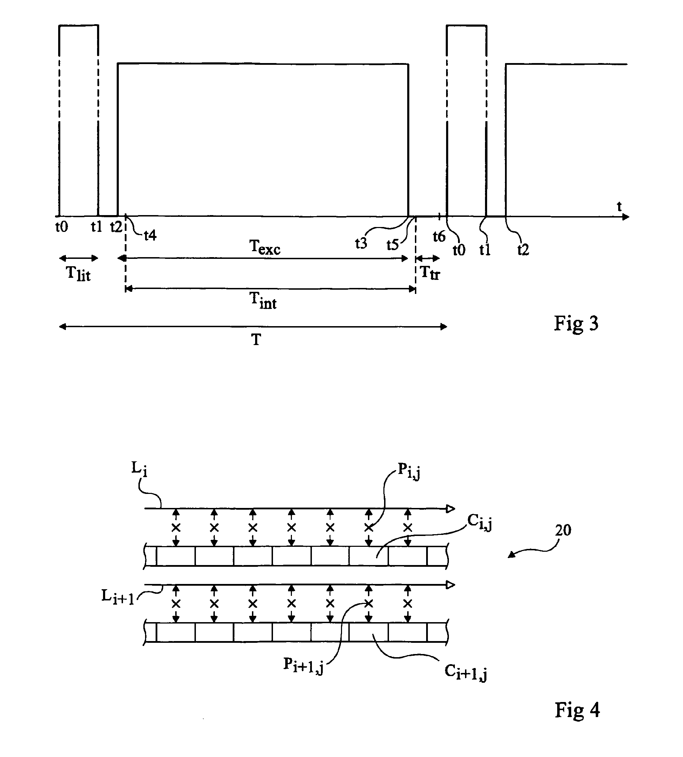

Le fonctionnement du système de la figure 2 va être expliqué en relation avec la figure 3. La figure 3 représente en abscisses le temps t et en ordonnées des intensités lumineuses. Dans ce qui suit, on suppose que les différents phénomènes décrits se déroulent à une périodicité T. Cette périodicité est choisie pour que, en raison du phénomène de persistance rétinienne, l'observateur d'éclairs de lumière blanche ait une impression d'éclairement continu. La cadence d'éclairement sera supérieure à 24/s, de préférence de l'ordre de 100/s.

La source lumineuse 2 d'éclairement ambiant, à faible rémanence, est éclairée pendant des durées Tₗᵢₜ entre des instants t0 et t1. Le rapport cyclique est relativement faible, c'est-à-dire que si, par exemple, la période T est de l'ordre de 10 millisecondes (fréquence de 100 Hz) l'allumage aura de préférence une durée non supérieure à 1 à 2 ms. A l'instant t1 de fin de l'impulsion d'éclairement, on suppose que l'intensité lumineuse chute très rapidement, en une durée non visible sur la figure par rapport à la durée d'éclairement, par exemple 1 à 3 µs.

Entre l'instant t1 de fin d'éclairement et l'instant t0 de début d'éclairement suivant de la source lumineuse faiblement rémanente fournissant la lumière d'ambiance, on procède à l'analyse de la fluorescence de la zone éclairée. Ainsi, pendant une durée d'excitation T_{exc} entre des instants t2 et t3, on éclaire, à l'aide de la source lumineuse 16 le champ à observer et, pendant cette durée ou pendant une durée Tᵢₙₜ décalée de quelques microsecondes, on intègre la lumière reçue par la caméra 7. On notera qu'il importe que la durée d'intégration se termine avant le début de l'éclairage suivant mais que, par contre, il n'est pas essentiel que la lumière d'excitation soit interrompue. Il pourrait s'agir d'une lumière continue. Comme on l'a indiqué précédemment, chaque période T comprendra une durée d'éclairement Tₗᵢₜ de la source lumineuse plus faible que la durée Tᵢₙₜ durant laquelle on collecte la lumière de fluorescence. Ainsi, si la période T est de l'ordre de 10 millisecondes, la durée Tₗᵢₜ pourra être inférieure à 2 ms et la durée Tᵢₙₜ pourra être supérieure à 6 ms.

Ensuite, entre l'instant t5 de fin d'intégration et un instant t6, pendant une durée Tₜᵣ, on transfère les informations recueillies par la caméra dans une mémoire.

Si l'intensité de la fluorescence est particulièrement faible, on pourra avoir besoin de plusieurs périodes d'intégration Tᵢₙₜ et de plusieurs transferts successifs avant d'avoir une image observable. Ceci est parfaitement compatible avec le système décrit.

Ainsi, selon le mode de réalisation décrit, on prévoit de dissocier les périodes d'éclairement d'ambiance des périodes d'analyse de la fluorescence.

Les diodes électroluminescentes ou autre sources lumineuses à faible rémanence excitables par impulsions périodiques fournissant l'éclairement d'ambiance seront choisies en nombre suffisant et avec des puissances suffisantes pour pouvoir éclairer de façon satisfaisante une salle d'opération et plus particulièrement un champ d'opération même si elles sont excitées par impulsions avec des rapports cycliques de l'ordre de 1 à 2/10.

A titre de variante, au lieu de prévoir des sources d'excitation spécifiques 16 pour exciter les éléments fluorescents, on pourra prévoir que ces sources sont constituées d'une partie des diodes électroluminescentes éclairant la salle d'opération.

La figure 4 illustre un mode de réalisation d'une partie d'un capteur d'image 20 susceptible d'être intégré à la caméra sensible à la fluorescence 7. On a représenté dans cette figure des pixels P_{i,j}, P_{i+1,j} par des croix. Les pixels P_{i,j} correspondent à une partie des pixels d'une ligne d'un capteur d'image matriciel, et les pixels P_{i+1,j} correspondent aux pixels d'une ligne adjacente du même capteur d'image. Chaque pixel P_{i,j} peut être connecté par un premier interrupteur à une ligne de vidange ou de décharge Li, symbolisée par une connexion à un potentiel de référence. Un deuxième interrupteur permet de connecter chaque pixel P_{i,j} à une cellule mémoire respective Cᵢ. Dans un mode réalisation usuel, les transferts sont effectués par ligne de pixels. Avant chaque début de période d'intégration Tᵢₙₜ (instant t4 de la figure 3), on connectera tous les pixels aux lignes de décharge pour les vider des charges créées par l'illumination. De préférence la connexion entre les pixels et les lignes de décharge est maintenue pendant toute la durée Tₗᵢₜ. Après cela seulement, l'intensité lumineuse de fluorescence incidente permettra une accumulation de charges au niveau de chacun des pixels en fonction de la lumière de fluorescence que ce pixel reçoit et, à la fin de chaque période, les informations stockées dans chaque ligne de pixel seront déchargées vers les lignes de cellules mémoire Cᵢ, Cᵢ₊₁. Ce mode de mise en oeuvre est particulièrement simple. Il permet d'accumuler des charges collectées au cours d'une multitude de périodes Tᵢₙₜ, par exemple 5 à 10 périodes Tᵢₙₜ, avant de procéder à la lecture de l'imageur matriciel et au transfert de l'image obtenue vers des moyens de traitement et de visualisation 10. Ce mode de mise en oeuvre permet d'accumuler le signal de fluorescence tout en réduisant l'importance relative du bruit de lecture. Selon ce mode de mise en oeuvre, l'imageur peut être une matrice CCD à transfert interligne tel le CCD KAI 340 fabriqué par Kodak, ou le CCD KAI 340 de Sony.

De nombreuses autres mises en oeuvre seront possibles, du moment que l'on prévoit un capteur d'image susceptible d'acquérir une image ou une partie d'une image après la fin d'une impulsion d'éclairement et de transférer cette image vers une mémoire avant le début de l'impulsion d'éclairement suivante.

On pourra aussi prévoir un obturateur, tel un obturateur mécanique ou un obturateur à cristaux liquides, qui occulte périodiquement le capteur d'image de fluorescence pendant les impulsions de lumière blanche, le capteur d'image pouvant être dans ce cas un capteur de type CCD ou CMOS. Ainsi, l'accumulation de la lumière de fluorescence sera prolongée sur le capteur, sa durée pouvant atteindre par exemple 5 à 10 périodes Tᵢₙₜ, avant d'être transférée vers des moyens de traitement de visualisation 10.

Dans le cas où la longueur d'onde centrale de la source d'excitation 16 des éléments fluorescents est dans le domaine du visible, la lumière fournie par cette source est intégrée par l'oeil de l'observateur au cours de chaque période avec la lumière de la source lumineuse d'ambiance 12. Il peut en résulter que la zone éclairée par la source d'excitation de fluorescence 16 apparaît avec une couleur et/ou un contraste différent en observation naturelle des autres zones de la salle ou autre lieu dans lequel se trouve le champ d'observation. Selon une variante de l'invention, pour compenser cet effet, on pourra prévoir que les sources 16 d'excitation sont en fait des sources doubles et comportent d'une part des sources telles que celles décrites précédemment propres à exciter les éléments fluorescents entre les périodes d'éclairement de la lumière d'ambiance, d'autre part des sources lumineuses complémentaires spectralement des sources d'excitation. Les sources complémentaires sont excitées pendant au moins une partie de la durée pendant laquelle les sources lumineuses d'excitation sont éteintes en dehors des périodes T_{exc}, Tᵢₙₜ et Tₜᵣ. Ces sources complémentaires sont excitées avec une intensité suffisante pour que, en raison de la persistance rétinienne, par intégration oculaire, la lumière résultante corresponde à la lumière d'ambiance ou à une couleur choisie pour le champ d'observation.

La présente invention est susceptible de nombreuses autres variantes qui apparaîtront à l'homme de l'art. Notamment, on pourra utiliser comme lumière d'ambiance toute source lumineuse excitable par impulsions et à très faible rémanence.

On rappellera que l'invention, bien que décrite dans le cadre d'une salle d'opération, s'applique de façon générale à tout système dans lequel on veut, d'une part, observer des objets à l'oeil nu en lumière "naturelle" et, d'autre part, observer par une caméra la lumière de fluorescence susceptible d'être fournie par des zones des objets en question.

L'homme de l'art notera également que l'on pourra prévoir que le champ d'observation 1 est susceptible de contenir plusieurs éléments fluorescents de caractéristiques différentes. On pourra alors prévoir d'analyser le champ d'observation par plusieurs caméras dont chacune est sensible à la lumière de l'un des éléments fluorescents. En ce cas, selon la distance entre les zones spectrales de fluorescence des différents éléments fluorescents, on pourra procéder à des analyses simultanées ou successives des éléments fluorescents par les diverses caméras.

Dans les modes de réalisation décrits ci-dessus, on a prévu d'afficher en superposition avec une image de fluorescence une image "naturelle". Pour ce faire, plusieurs caméras pourront viser le même champ d'observation sans utiliser de séparatrice. Dans certains cas, l'homme de l'art pourra ne pas afficher l'image naturelle mais seulement l'image de fluorescence. De plus, l'image de fluorescence pourra être superposée à une image fixe, par exemple un dessin ou une radiographie. On pourra aussi prévoir que la même caméra détecte alternativement l'image naturelle et l'image de fluorescence.

## Revendications

1. Système d'analyse à l'oeil nu et par fluorescence d'un champ dans une zone éclairée comprenant :
une première source lumineuse d'éclairement (12) en lumière blanche à rémanence de l'ordre de quelques microsecondes constituée d'un assemblage de diodes électroluminescentes excitées périodiquement à une fréquence supérieure à 24 Hz, ladite rémanence étant faible devant la période d'excitation de la première source lumineuse ;
une deuxième source lumineuse (16) d'excitation d'éléments fluorescents situés dans ledit champ, active au moins pendant une partie des durées d'extinction de la première source ; et
un dispositif d'analyse de fluorescence (7) actif pendant des durées d'extinction de la première source et d'allumage de la deuxième source, la période d'éclairement (T) de la première source d'éclairement comprenant une première durée d'éclairement (Tlit) de la première source lumineuse (16), et une deuxième durée (Tint) durant laquelle on collecte la lumière de fluorescence, la première durée (Tlit) étant inférieure à la deuxième durée (Tint).

2. Système selon la revendication 1, dans lequel ladite fréquence d'excitation périodique est de l'ordre de 100 Hz.

3. Système selon la revendication 1 ou 2, dans lequel le rapport cyclique d'excitation de la première source lumineuse est inférieur à 2/10.

4. Système selon la revendication 1, dans lequel la deuxième source lumineuse fournit de la lumière dans une région spectrale ne recouvrant pas le spectre d'émission des éléments fluorescents.

5. Système selon la revendication 1, dans lequel le dispositif d'analyse est une caméra électronique.

6. Système selon la revendication 5, dans lequel la caméra électronique comprend une matrice de pixels à transfert par ligne.

7. Système selon la revendication 5, dans lequel l'image de ladite caméra électronique est superposée à l'image fournie par une autre caméra électronique observant ledit champ à la lumière de la première source lumineuse d'éclairement (12).

8. Système selon l'une quelconque des revendications 1 à 7, comprenant une troisième source lumineuse de spectre sensiblement complémentaire de celui de la deuxième source lumineuse, éclairant sensiblement le même champ et active de façon complémentaire de la deuxième source lumineuse.

9. Système selon l'une quelconque des revendications 1 à 7, dans lequel la deuxième source lumineuse (16) d'excitation d'éléments fluorescents est continue.

## Patentansprüche

1. Ein System zur Analyse mit nacktem Auge und durch Fluoreszenz eines Feldes in einem beleuchtetem Gebiet wobei Folgendes vorgesehen ist:
eine erste Weißlichtbeleuchtungsquelle (12) mit einer Remanenz in der Größenordnung von einigen Mikrosekunden ausgebildet aus einer Anordnung von Licht emittierenden Dioden die periodisch angeregt werden und zwar mit einer Frequenz größer als 24 Hz, wobei die Remanenz kurz bezüglich der Anregungsperiode der ersten Lichtquelle ist;
eine zweite Lichtquelle (16) zur Anregung der Fluoreszenzelemente angeordnet in dem erwähnten Feld und zwar aktiv mindestens während eines Teils der Zeitperioden wenn die erste Quelle ausgeschaltet ist; und
eine Fluoreszenzanalysevorrichtung (7) aktiv während Zeitperioden wenn die erste Quelle ausgeschaltet ist und die zweite Quelle eingeschaltet ist, wobei die Einschaltperiode (T) der ersten Lichtquelle eine erste Beleuchtungsdauer (Tlit) der ersten Lichtquelle (16) und eine zweite Belichtungsdauer (Tint) während des Sammelns des Fluoreszenzlichtes ist, wobei die erste Dauer (Tlit) kleiner ist als die zweite Dauer (Tint).

2. System nach Anspruch 1, wobei die periodische Erregungsfrequenz in der Größenordnung von 100 Hz liegt.

3. System nach Anspruch 1 oder 2, wobei der Ansteuergrad des einen Zustandes der ersten Lichtquelle kleiner ist als 2/10.

4. System nach Anspruch 1, wobei die zweite Lichtquelle Licht in einem Spektralbereich liefert, der das Emissionsspektrum der Fluoreszenzelemente nicht überdeckt.

5. System nach Anspruch 1, wobei die Analysevorrichtung eine elektronische Kamera ist.

6. System nach Anspruch 5, wobei die elektronische Kamera eine Zeilen- bzw. Line-Transferpixelanordnung ist.

7. System nach Anspruch 5, wobei das Bild der elektronischen Kamera dem Bild überlagert wird welches durch eine weitere elektronische Kamera erzeugt wird, die das erwähnte Feld durch das Licht der ersten Beleuchtungslichtquelle (12) beobachtet.

8. System nach einem der Ansprüche 1 bis 7, wobei Folgendes vorgesehen ist:
eine dritte Lichtquelle mit einem Spektrum im Wesentlichen komplementär zu dem der zweiten Lichtquelle und zwar im Wesentlichen das gleiche Feld beleuchtend, und die zur zweiten Lichtquelle komplementär aktiv ist.

9. System nach einem der Ansprüche 1 bis 7, wobei die zweite Lichtquelle (16) zur Erregung der Fluoreszenz der Elemente kontinuierlich arbeitet.

## Claims

1. A system of analysis with the naked eye and by fluorescence of a field in an illuminated area comprising:
a first white light illumination source (12) with a remanence of the order of some microseconds formed of an assembly of light-emitting diodes periodically excited at a frequency greater than 24 Hz, said remanence being short with respect to the excitation period of the first light source;
a second light source (16) for exciting fluorescent elements located in said field, active at least during part of the time periods when the first source is off; and
a fluorescence analysis device (7) active during time periods when the first source is off and the second source is on, the on period (T) of the first light source comprising a first lighting duration (Tlit) of the first light source (16) and a second time duration (Tint) during which the fluorescence light is collected, the first duration (Tlit) being smaller than the second duration (Tint).

2. The system of claim 1, wherein said periodic excitation frequency is on the order of 100 Hz.

3. The system of claim 1 or 2, wherein the duty cycle of the on-state of the first light source is smaller than 2/10.

4. The system of claim 1, wherein the second light source provides light in a spectral region which does not cover the emission spectrum of the fluorescent elements.

5. The system of claim 1, wherein the analysis device is an electronic camera.

6. The system of claim 5, wherein the electronic camera comprises a line-transfer pixel array.

7. The system of claim 5, wherein the image of the electronic camera is superposed to the image provided by another electronic camera observing said field by the light of the first illumination light source (12).

8. The system of any of claims 1 to 7, comprising a third light source having a spectrum substantially complementary to that of the second light source, substantially illuminating the same field, and which is active complementarily to the second light source.

9. The system of any of claims 1 to 7, wherein the second light source (16) of excitation of fluorescent elements is continuous.
